# EUROPÄISCHE PATENTSCHRIFT

(11) **EP 2 329 800 B1**
(45) Veröffentlichungstag und Bekanntmachung des Hinweises auf die Patenterteilung: **29.02.2012**
(21) Anmeldenummer: 10014768.5
(22) Anmeldetag: 19.11.2010
(51) Int. Cl.: A61F 5/01

(54) **Kniegelenkorthese**
Knee orthotic
Orthèse du genou

(30) Priorität: 07.12.2009 DE 102009057700
(43) Veröffentlichungstag der Anmeldung: 08.06.2011
(73) Patentinhaber: Otto Bock HealthCare GmbH, 37115 Duderstadt (DE)
(72) Erfinder: Vollbrecht, Matthias, 37412 Herzberg am Harz (DE); Brüggemann, Gert-Peter, Prof. Dr., 50858 Köln (DE); Gösele- Koppenburg, Andreas, 79540 Lörrach (DE); Best, Raymond, Dr., 70180 Stuttgart (DE); Ellermann, Andree, Dr., 76275 Ettlingen (DE); Semsch, Hartmut, 70374 Stuttgart (DE); Albasini, Alfio, 6595 Riazzino Ticino (CH); Liebau, Christian, Dr., 38126 Braunschweig (DE); Petersen, Wolf, Prof. Dr., 14109 Berlin (DE)
(74) Vertreter: Lins, Edgar

(56) Entgegenhaltungen:
- WO-A2-2004/069109
- US-A- 5 807 298
- US-B2- 7 059 329
- US-B2- 7 060 045

## Beschreibung

Die Ereindung betrifft eine Kniegetenkorthese zur Führung der Patella eines Patienten beim Übergang einer gestreckten in eine gebeugte Stellung des Kniegelenks und umgekehrt, mit einer Befestigungseinrichtung zur Befestigung an einem Oberschenkel und einem Unterschenkel, mit wenigstens einem lateral angeordneten, eine Beugung des Kniegelenks aufnehmenden Stabilisierungselement zur Anlage am Oberschenkel und Unterschenkel und mit einem durch eine Spannbandanordnung in Position haltbaren formstabilen Führungselement, das zum lateralen Anliegen an der Patella mittels eines seitlichen Basisteils angeordnet ist, von dem sich nach medial gerichtete Ansätze proximal und distal von der Patella erstrecken und mit Bandabschnitten an der Orthese im Bereich des Oberschenkels und des Unterschenkels so befestigt sind, dass ein medialer Zug auf die Ansätze ausgeübt wird, wobei die Bandabschnitte mit einer Elastizität ausgebildet sind und mit dem Basisteil ein lateral erstrecktes, eine seitliche Verschiebung des Basisteils erlaubendes elastisches Band verbunden ist.

Derartige Kniegelenkorthesen, die der Führung der Patella (Kniescheibe) dienen, werden benötigt, wenn aufgrund von Abnormitäten oder Schädigungen die Führung der Patella im Tibiakopf, insbesondere in der gestreckten Stellung des Kniegelenks, nicht mehr gewährleistet ist. Kommt es dann zu einer Verschiebung der Patella, entstehen zumindest beim anschließenden Beugen des Kniegelenks schmerzhafte Fehlstellungen der Patella. Eine Kniegelenkorthese der eingangs erwähnten Art hat die Aufgabe, die Patella auch dann in der richtigen Position zu halten, wenn eine Führung der Patella im Kniegelenk selbst nicht über den gesamten Bewegungsbereich des Kniegelenks gewährleistet ist.

Durch US 7,059,329 B2 bzw. US 7,060,045 B2 sind derartige Kniegelenkorthesen bekannt. Sie bestehen aus Oberschenkel-Formteil und einem Unterschenkel-Formteil, die mittels mit Ihnen verbundenen Schienen über zwei seitliche Gelenke miteinander schwenkbar verbunden sind. Das C-förmige Führungselement ist mit seinen beiden Ansätzen, die die Patella distal und proximal teilweise umgreifen, über Spannriemen mit dem Gelenk auf der einen Seite verbunden, während das Basisteil des Führungselements über einen in seiner Länge einstellbaren Riemen mit dem anderen Gelenk verbunden ist. Das C-förmige Führungselement weist ein Polster auf, mit dem es nicht nur seitlich von der Patella anliegt, sondern den vorzugsweise lateralen Rand der Patella auch von oben beaufschlagt, sodass die Patella gegen das Kniegelenk gedrückt wird. Ein derartiger Druck auf die Patella wird von dem Orthesenträger nach einer gewissen Zeit als unangenehm empfunden. Die Orthese ist in Verbindung mit einer über das Kniegelenk gezogenen Hülse aus elastischem Material vorgesehen, wobei die Hülse eine Öffnung für die Patella aufweist. ,

Eine Kniegelenkorthese der eingangs erwähnten Art, deren Aufbau den vorbeschriebenen Kniegelenkorthesen ähnelt, ist durch US 5,807,289 bekannt. Das Basisteil des Führungselements ist dabei mit einer zentralen Riemenanordnung verbunden, an die sich elastische Riemenanordnungen anschließen, sodass das Basisteil des Führungselements mit einer Vorspannung auf die Patella gedrückt wird.

Durch WO 2004/069109 A2 ist eine Orthese bekannt, die keine Gelenkschienen aufweist sondern die Führung der Patella durch eine in geeigneter Weise ausgebildete Hülse aus elastischem Material bewirkt. Seitlich von einer Öffnung für die Patella befindet sich dabei ein Andruckpolster, das mit elastischen Spannbändern gegen das Kniegelenk und den Rand der Patella gedrückt wird. Auch hier besteht das Problem des Tragekomforts durch das Drücken von Oben auf die Patella. Darüber hinaus fehlt es an einer Führung durch ein seitliches Gelenk mit Gelenkschienen.

Der vorliegenden Erfindung liegt die Aufgabe zugrunde, eine Kniegelenkorthese der eingangs erwähnten Art so auszubilden, dass sie für eine zuverlässige Führung der Patella beim Beugen und Strecken des Kniegelenks sorgt und verbesserte Trageigenschaften aufweist.

Diese Aufgabe wird erfindungsgemäß mit einer Kniegelenkorthese der eingangs erwähnten Art dadurch gelöst, dass die Elastizität des lateral erstreckten Bandes größer ist als die Elastizität der Bandabschnitte und dass die Elastizitäten der medial erstreckten Bandabschnitte einerseits und des lateral erstreckten Bandes anderseits so gewählt sind, dass bei einer Beugung des Gelenks aus der gestreckten Stellung sich zunächst zumindest ganz überwiegend das lateral erstreckte Band dehnt und dass ein Dehnungsbegrenzer für das lateral erstreckte Band vorgesehen ist, der bei einem vorgegebenen Beugungsgrenzwinkel des Kniegelenks wirksam ist, sodass sich für eine größere Beugung des Kniegelenks die medial erstreckten Bandabschnitte zumindest überwiegend denen.

Bei der erfindungsgemäßen Kniegelenkorthese wird das Führungselement einerseits neben der Patella gehalten, sodass sie nicht von oben auf die Patella drücken kann, und andererseits auch bei größeren Beugungswinkeln ein zur starker Druck auf die Patella vermieden. Wenn das Knie aus der gestreckten Lage gebeugt wird, wird das im gestreckten Zustand lateral an der Patella anliegende Führungselement zur Führung der Patella etwas in Richtung auf die Patella verschoben, bis ein Beugungsgrenzwinkel erreicht wird. Durch den Dehnungsbegrenzer wird die seitliche Verschiebung des Führungselements aufgrund der ursprünglichen Dehnung des lateral erstreckten Bandes zumindest weitgehend unterbunden, sodass die für die weitere Beugung benötigte Elastizität durch die medial erstreckten Bandabschnitte zur Verfügung gestellt wird. Dadurch ändert sich die Position des Führungselements relativ zur Patella praktisch nicht mehr, wenn der Beugungsgrenzwinkel überschritten wird. Das lateral erstreckte Band ist in der Höhe des Gelenks vorgesehen, sodass es sich beim Beugen oder Strecken des Kniegelenks nicht merklich in Längsrichtung verschiebt.

Bevorzugt ist die Ausführungsform, bei der die Dehnungsbegrenzung bei der Überschreitung des Beugungsgrenzwinkels eine weitere Dehnung des lateral erstreckten Bandes vollständig unterdrückt, sodass sich nur noch die medial erstreckten Bandabschnitte dehnen.

In einer bevorzugten Ausführungsform der Erfindung kann die Kniegelenkorthese mit einer Hülse aus elastischem Material kombiniert sein, die im Kniebereich über das Bein des Patienten gezogen wird und dort elastisch anliegt. Die Hülse weist vorzugsweise ein frontales Loch auf, durch das die Patella ragen kann, sodass ein radial nach innen gerichteter Druck durch die Patella durch die Hülse vermieden wird. Die Schienen des seitlichen Gelenks können an der Hülse befestigt sein, sodass die Hülse als Befestigungseinrichtung für die Kniegelenkorthese fungiert.

Das lateral vorgesehene Stabilisierungselement, das die Beugebewegung des Kniegelenks am Oberschenkel und Unterschenkel aufnehmen kann, kann ein Federelement sein. Bevorzugt ist jedoch eine Ausführungsform, bei der lateral ein Gelenk vorgesehen ist, durch das eine Oberschenkelschiene am Oberschenkel und eine Unterschenkelschiene am Unterschenkel so anliegt, dass die relative Bewegung von Oberschenkel und Unterschenkel über eine entsprechende Bewegung der Schienen als Drehbewegung in dem Gelenk aufgenommen wird. Das Gelenk kann an der Hülse aus elastischem Material befestigt sein.

Das lateral erstreckte Band kann dann direkt an dem lateral angeordneten Gelenk befestigt werden. Alternativ hierzu kann das lateral erstreckte Band auch an der Hülse selbst befestigt werden, indem es mit Klettverschlusselementen an der entsprechend ausgebildeten Hülse unmittelbar festgelegt wird. In diesem Fall erübrigt sich eine längenverstellbare Ausbildung des lateral erstreckten Bandes, während anderenfalls das Band vorzugsweise mit einem längenverstellbaren Abschnitt versehen ist. In diesem Fall kann das lateral erstreckte Band sowohl einen nichtelastischen, längenverstellbaren Abschnitt als auch einen elastischen Abschnitt aufweisen.

Die erfindungsgemäße Kniegelenkorthese kann auch auf der medialen Seite mit einem Gelenk mit zwei Schienen ausgebildet sein, die die Bewegung von Oberschenkel und Unterschenkel im Bereich des Kniegelenks aufnehmen.

Die Elastizität der medial erstreckten Bandabschnitte kann durch eine Materialelastizität zur Verfügung gestellt werden. In einer bevorzugten Ausführungsform kann eine präzisere Elastizität der Bandabschnitte dadurch eingestellt werden, dass diese in die Stützschienen hineingeführt werden und dort mit einem in einen Schlitz der Stützschiene gehaltenen Federelement verbunden sind. Die Elastizität des Bandabschnitts ergibt sich dann durch das Federelement.

Die medial erstreckten Bandabschnitte sind vorzugsweise längenverstellbar ausgebildet, um eine Anpassung der Kniegelenkorthese an den Patienten zu ermöglichen.

Das lateral erstreckte Band weist eine höhere Elastizität auf als die medial erstreckten Bandabschnitte, ist also viel leichter dehnbar. Bei einer Beugung des Gelenks aus dem gestreckten Zustand wird also zunächst ganz überwiegend das lateral erstreckte Band gedehnt. Parallel zu dem lateral erstreckten Band wird das Führungselement beispielsweise durch ein im Wesentlichen unelastisches Dehnungsbegrenzungsband gehalten, das im gestreckten Zustand des Gelenks eine überschüssige Länge aufweist, sodass durch das Dehnungsbegrenzungsband im gestreckten Zustand keine Zugwirkung auf das Führungselement ausgeübt wird. Wenn sich beim Beugen des Gelenks bis zum Beugungsgrenzwinkel das lateral erstreckte elastische Band so weit gedehnt hat, dass nunmehr auch das Dehnungsbegrenzungsband eine Zugwirkung auf das Führungselement ausübt, verhindert die zumindest weitgehende Unelastizität des Dehnungsbegrenzungsbandes ein weiteres Dehnen des lateral erstreckten elastischen Bandes, sodass die für die weitere Beugung des Gelenks erforderliche Elastizität durch die elastischen Bandabschnitte zur Verfügung gestellt wird. Aufgrund der geringeren Elastizität der medial erstreckten Bandabschnitte gegenüber der Elastizität des lateral erstreckten Bandes wird der weiteren Beugungsbewegung dabei ein höherer Widerstand entgegengesetzt. Alternativ kann die Dehnungsbegrenzung in das lateral erstreckte Band eingearbeitet sein, beispielsweise durch aufgesetzte Zick-Zack-Nähte, eingewobene oder eingestrickte Fäden o. dgl.

Die Befestigung der medial erstreckten Bandabschnitte an der Knieorthese muss so erfolgen, dass ein medialer Zug auf die medial gerichteten Ansätze des Führungselements bei der Beugung des Kniegelenks ausgeübt wird. Demzufolge ist eine der Bandabschnitte am Oberschenkel und der andere der Bandabschnitte am Unterschenkel befestigt. Eine unmittelbare mediale Zugeinleitung kann dadurch erfolgen, dass die Bandabschnitte im medialen Bereich oder im dorsalen Bereich befestigt sind. Es ist aber auch eine Befestigung im frontalen Bereich möglich, wenn eine entsprechende Umlenkung der Bandabschnitte erfolgt, wodurch die beim Beugen nach proximal und distal gerichtete Zugkraft in eine medial gerichtete Zugkraft umgelenkt wird. Insbesondere hierzu können die Bandabschnitte - zumindest teilweise - auch schnur- oder drahtförmig ausgebildet sein.

Die Erfindung soll im Folgenden anhand eines in der Zeichnung dargestellten Ausführungsbeispiels näher erläutert werden. Es zeigen:
- Figur 1: eine perspektivische Ansicht einer erfindungsgemäßen Knieorthe- se schräg von der lateralen Seite;
- Figur 2: eine perspektivische Ansicht der Knieorthese gemäß Figur 1 schräg von der medialen Seite;
- Figur 3: eine schematische Darstellung des Führungselements mit dem lateral erstreckten Band und den medial erstreckten Bandabschnit- ten und der Führungsschienen des Gelenks im gestreckten Zu- stand;
- Figur 4: eine Darstellung gemäß Figur 3 in einem um einen Beugungs- grenzwinkel gebeugten Zustand des Gelenks;
- Figur 5: eine Darstellung gemäß Figur 3 für das über den Beugungsgrenz- winkel hinaus gebeugte Gelenk.

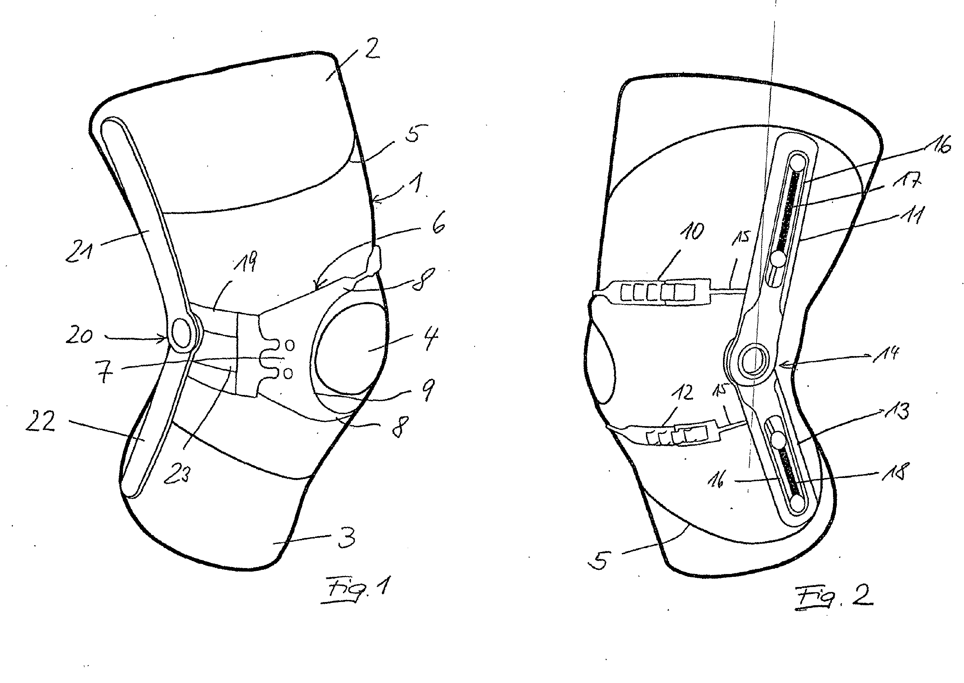

Die Figuren 1 und 2 zeigen in einer schematischen Darstellung eine Kniegelenkorthese für ein rechtes Kniegelenk eines Patienten. Die Orthese weist eine über das Kniegelenk zu ziehende und an die Form eines Kniegelenks angepasste Hülse 1 aus einem textilen elastischen Material auf. Die Hülse bildet einen Oberschenkelabschnitt 2 und einen Unterschenkelabschnitt 3. Die Hülse 1 weist an ihrer Vorderseite eine Öffnung 4 auf, durch die bei korrekt angelegter Orthese eine Patella eines Patienten ragt. Die Hülse 1 ist aus mehreren über Nähte 5 miteinander verbundenen Materialstücken gebildet.

Auf der Hülse 1 liegt lateral neben der Öffnung ein formstabiles Führungselement 6 an, das aus einem lateral neben der Öffnung 4 liegenden Basisteil 7 und sich von dort nach medial erstreckenden Ansätzen 8 besteht, die oberhalb und unterhalb der Öffnung 4 verlaufen, sodass das Führungselement 6 etwa C-förmig ausgebildet ist. Ein entsprechender C-förmiger Rand 9 kann vom Basisteil 7 aus nach oben hochgebogen und ggf. mit einer Polsterung versehen sein, um ein sanftes Anliegen am Rand der Patella zu ermöglichen.

Der obere Ansatz 8 ist über einen Bandabschnitt 10 mit einer Oberschenkelschiene 11 und der untere Ansatz 8 über einen Bandabschnitt 12 mit einer Unterschenkelschiene 13 eines ersten Gelenks 14 verbunden. Die Bandabschnitte 10, 12 sind längenverstellbar ausgebildet und ragen mit einem schnurähnlichen Ende 15 in die Schienen 11, 13 hinein, wo sie mit einem in einen Schlitz 16 der Schienen 11, 13 geführten Federelement 17, 18 verbunden sind. Die Bandabschnitte 10, 12 sind somit durch die Federelemente 17, 18 elastisch ausgebildet, wobei das Material außerhalb der Federelemente 17, 18 unelastisch sein kann, sodass sich die Elastizität nur aus der Elastizität der Federelemente 17, 18 ergibt.

An das Basisteil 7 des Führungselements 6 schließt sich lateral ein Band 19 an, das aus einem elastischen Material besteht. Das Band 19 stellt die Verbindung zwischen dem Führungselement 6 und einem zweiten Gelenk 20 her, mit dem das Band 19 an seinem zweiten Ende verbunden ist. Das zweite Gelenk 20 weist ebenfalls eine Oberschenkelschiene 21 und eine Unterschenkelschiene 22 auf, die beide mit der Hülse 1 verbunden sind, beispielsweise durch Einschieben in entsprechende Taschen der Hülse 1.

Das Band 19 ist durch ein Dehnungsbegrenzungsband 23 überbrückt, das aus einem unelastischen Material besteht und ebenfalls eine Verbindung zwischen dem zweiten Gelenk 20 und dem Führungselement 6 herstellt.

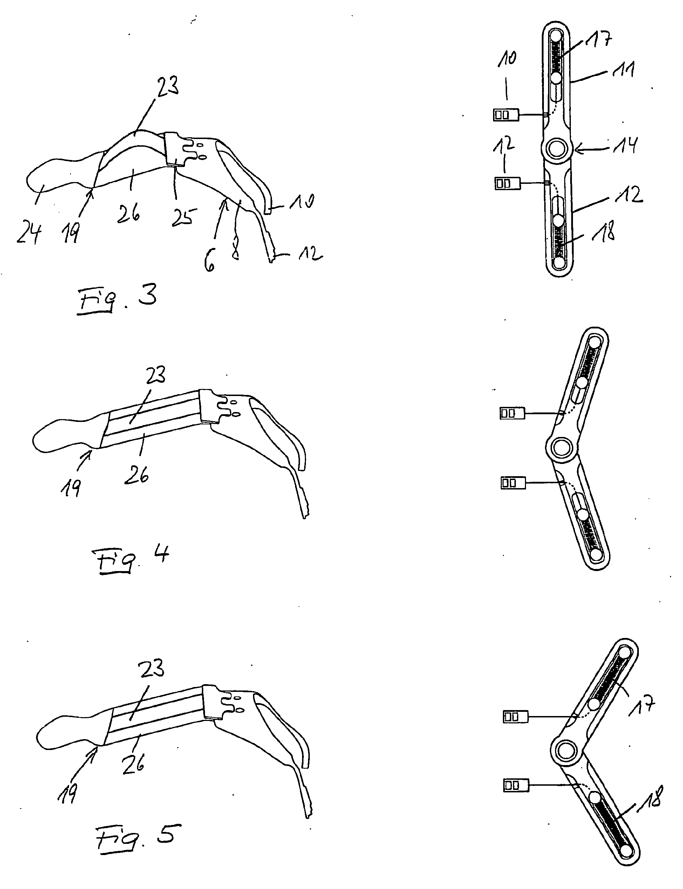

Die Figuren 3 bis 5 lassen erkennen, dass das lateral erstreckte Band 19 aus Befestigungsabschnitten 24, 25 besteht, zwischen denen ein elastischer Abschnitt 26 angeordnet ist. Der Befestigungsabschnitt 24 dient zur Befestigung des Bandes 19 an dem zweiten Gelenk 20 und der Befestigungsabschnitt 25 zur Befestigung des Bandes 19 an dem Führungselement 6. Figur 3 lässt erkennen, dass der elastische Abschnitt 26 des Bandes 19 durch das Dehnungsbegrenzungsband 23 überbrückt ist, dass somit ebenfalls zwischen dem Befestigungsabschnitten 24, 25 angeordnet ist. Das Dehnungsbegrenzungsband 23 hat eine größere Länge als der elastische Abschnitt 26 in der gestreckten Stellung des Gelenks 14, sodass sich das Dehnungsbegrenzungsband 23 aufgrund seiner Überlänge nach oben wölbt, wie dies in Figur 3 dargestellt ist. Figur 3 lässt ferner erkennen, dass die Federelemente 17, 18 durch die Bänder 10, 12. sich im gestreckten Zustand in einer Ausgangsstellung befinden, in der sie nicht oder nur wenig gedehnt sind.

Wird das Kniegelenk - und damit das Gelenk 14 - bis zu einem Beugungsgrenzwinkel gebeugt, wie er in Figur 4 dargestellt ist, dehnt sich der elastische Abschnitt des Bandes 19, weil der elastische Abschnitt 26 eine wesentlich höhere Elastizität aufweist als die Federelemente 17, 18, die in der Beugung bis zum Beugungsgrenzwinkel praktisch nicht gedehnt werden. Durch die Längen des elastischen Abschnitts 26 streckt sich das unelastische Dehnungsbegrenzungsband 23, das beim Erreichen des Beugungsgrenzwinkels gemäß Figur 4 beginnt, auf Zug belastet zu werden.

Wird das Kniegelenk - und damit das Gelenk 14 - weiter gebeugt, wie dies in Figur 5 dargestellt ist, verhindert das Dehnungsbegrenzungsband 23 eine weitere Dehnung des elastischen Abschnitts 26 des Bandes 19, sodass die für die Beugung erforderliche Elastizität nunmehr durch die Dehnung der Federelemente 17, 18 bereitgestellt wird.

Für das Führungselement bedeutet dies, dass das Führungselement bei einer Beugung aus der gestreckten Stellung der Figur 3 in die bis zum Beugungsgrenzwinkel gebeugte Stellung gemäß Figur 4 eine leichte seitliche Verschiebung nach medial erfährt, weil der elastische Abschnitt 26 sich aufgrund seiner hohen Elastizität längt. Bei einer weiteren Beugung verhindert das Dehnungsbegrenzungsband 23 eine weitere Längung des elastischen Abschnitts 26, sodass das Führungselement 6 nicht mehr seitlich verschoben wird, sondern seine Position lateral/medial während einer weiteren Beugung des Kniegelenks beibehält. Demgemäß längen sich die Bandabschnitte 10, 12 aufgrund der Dehnung ihrer Federelemente 17, 18.

Auch wenn in dem dargestellten Ausführungsbeispiel die Längung der Bandabschnitte 10, 12 über Federelemente 17, 18 beschrieben ist, stellt dies keine notwendige Lösung dar. Es ist ohne weiteres möglich, die Bandabschnitte 10, 12 aus einem elastischen Material zu bilden oder in die Bandabschnitte 10, 12 elastische Abschnitte - ähnlich wie der elastische Abschnitt 26 - einzusetzen. Wichtig ist dabei lediglich, dass die so gebildete Elastizität der Bandabschnitte 10, 12 deutlich geringer ist als die Elastizität des elastischen Abschnitts 26, also die Elastizität des lateral erstreckten Bandes 19. Für die erfindungsgemäße Funktion ist es wesentlich, dass zunächst nur oder überwiegend das lateral erstreckte Band 19 gedehnt wird und dass die seitliche Bewegung des Führungselements 6 dann durch das Dehnungsbegrenzungsband 23 gestoppt wird, sodass eine weitere seitliche Bewegung des Führungselements 6 bei einer weiteren Beugung des Gelenks 14 zu keiner oder zumindest keiner wesentlichen seitlichen Bewegung des Führungselements mehr führt.

Zur Klarstellung wird darauf hingewiesen, dass die Figuren 3 bis 5 den Vorgang bei der Beugung des Gelenks von der gestreckten Stellung der Figur 3 in die stark gebeugte Stellung der Figur 5 darstellen. Selbstverständlich gilt die Darstellung und die Funktionsbeschreibung auch für die Streckung des Gelenks aus der gebeugten Stellung der Figur 5 in die gestreckte Stellung der Figur 3, wobei die Führung der Patella durch das Führungselement 6 aus der vom Beugungsgrenzwinkel der Figur 4 gebildeten Stellung in die gestreckte Stellung besonders wichtig ist.

Der oben definierte Beugungsgrenzwinkel, bis zu dem eine seitliche Verschiebung des Führungselements 6 stattfindet, liegt bei etwa 30° Beugung des Kniegelenks.

Das Gelenk 14 und die damit verbundenen Schienen 11, 13 sind für die Steuerung des Führungselements 6 nicht essentiell und können daher in der Knieorthese auch entfallen. Ihre Funktion besteht lediglich in einer gewissen Erhöhung der Stabilität der Knieorthese, wenn die Bandabschnitte 10, 12 in anderer geeigneter Weise mit ihren freien Enden an der Knieorthese festgelegt werden, beispielsweise durch auf die Hülse 1 aufgesetzte Kfettverschlussabschnitte.

## Patentansprüche

1. Kniegelenkorthese zur Führung der Patella eines Patienten beim Übergang einer gestreckten in eine gebeugte Stellung des Kniegelenks und umgekehrt, mit einer Befestigungseinrichtung (1) zur Befestigung an einem Oberschenkel und einem Unterschenkel, mit wenigstens einem lateral angeordneten, eine Beugung des Kniegelenks aufnehmenden Stabilisierungselement zur Anlage am Oberschenkel und Unterschenkel und mit einem durch eine Spannbandanordnung in Position haltbaren formstabilen Führungselement (6), das zum lateralen Anliegen an der Patella mittels eines seitlichen Basisteils (7) angeordnet ist, von dem sich nach medial gerichtete Ansätze (8) proximal und distal von der Patella erstrecken und mit Bandabschnitten (10, 12) an der Orthese im Bereich des Oberschenkels und des Unterschenkels so befestigt sind, dass ein medialer Zug auf die Ansätze (8) ausgeübt wird, wobei die Bandabschnitte (10, 12) mit einer Elastizität ausgebildet sind und mit dem Basisteil (7) ein lateral erstrecktes, eine seitliche Verschiebung des Basisteils (7) erlaubendes elastisches Band (19) verbunden ist, **dadurch gekennzeichnet, dass** die Elastizität des lateral erstreckten Bandes (19) größer ist als die Elastizität der Bandabschnitte (10, 12) und dass die Elastizitäten der medial erstreckten Bandabschnitte (10, 12) einerseits und des lateral erstreckten Bandes (19) anderseits so gewählt sind, dass bei einer Beugung des Gelenks (14) aus der gestreckten Stellung sich zunächst zumindest ganz überwiegend das lateral erstreckte Band (19) dehnt und dass ein Dehnungsbegrenzer (23) für das lateral erstreckte Band (19) vorgesehen ist, der bei einem vorgegebenen Beugungsgrenzwinkel des Kniegelenks wirksam ist, sodass sich für eine größere Beugung des Kniegelenks (14) die medial erstreckten Bandabschnitte (10, 12) zumindest überwiegend dehnen.

2. Kniegelenkorthese nach Anspruch 1, **dadurch gekennzeichnet, dass** sich beim Überschreiten des Beugungsgrenzwinkels nur noch die medial erstreckten Bandabschnitte (10, 12) dehnen.

3. Kniegelenkorthese nach Anspruch 1 oder 2, **dadurch gekennzeichnet, dass** das lateral angeordnete Stabilisierungselement durch ein Gelenk (20) mit zwei durch das Gelenk (20) drehbar zueinander angebrachten Schienen (21, 22) gebildet ist, von denen eine Oberschenkelschiene (21) mit der Befestigungseinrichtung (1) am Oberschenkel und eine Unterschenkelschiene (22) mit der Befestigungseinrichtung (1) am Unterschenkel anliegend verbunden ist.

4. Kniegelenkorthese nach einem der Ansprüche 1 bis 3, **dadurch gekennzeichnet, dass** auf der medialen Seite ein Gelenk (14) mit zwei durch das Gelenk (14) drehbar zueinander angebrachten Schienen (11, 13) vorgesehen ist, von denen eine Oberschenkelschiene (11) mit der Befestigungseinrichtung (1) am Oberschenkel und eine Unterschenkelschiene (13) mit der Befestigungseinrichtung (1) am Unterschenkel anliegend verbunden ist.

5. Kniegelenkorthese nach Anspruch 4, **dadurch gekennzeichnet, dass** die medial erstreckten Bandabschnitte (10, 12) mit einem in der zugehörigen Schiene (11, 13) des medialen Gelenks (14) gehaltenen Federelement (17, 18) verbunden sind und dass sich die Elastizität der Bandabschnitte (10, 12) aus der Elastizität des zugehörigen Federelements (17, 18) ergibt.

6. Kniegelenkorthese nach einem der Ansprüche 1 bis 5, **dadurch gekennzeichnet, dass** die Befestigungseinrichtung durch eine zum Überziehen über Ober- und Unterschenkel ausgebildeten Hülse (1) aus elastischem Material gebildet ist.

7. Kniegelenkorthese nach einem der Ansprüche 1 bis 6, **dadurch gekennzeichnet, dass** das lateral erstreckte Band (19) mit Klettverschlusselementen auf der Hülse (1) befestigt ist.

8. Kniegelenkorthese nach einem der Ansprüche 3 bis 7, **dadurch gekennzeichnet, dass** das lateral erstreckte Band (19) an dem lateral angeordneten Gelenk (20) befestigt ist.

9. Kniegelenkorthese nach einem der Ansprüche 1 bis 8, **dadurch gekennzeichnet, dass** die medial erstreckten Bandabschnitte (10, 12) längenverstellbar ausgebildet sind.

10. Kniegelenkorthese nach einem der Ansprüche 1 bis 9, **dadurch gekennzeichnet, dass** das Führungselement (6) einen hochgebogenen Rand (9) zur Anlage an einer seitlichen Kante der Patella ausgebildet ist.

11. Kniegelenkorthese nach einem der Ansprüche 1 bis 10, **dadurch gekennzeichnet, dass** die medial erstreckten Bandabschnitte (10, 12) mit Klettverschlusselementen auf der Hülse (1) befestigt sind.

12. Kniegelenkorthese nach einem der Ansprüche 5 bis 10, **dadurch gekennzeichnet, dass** die medial erstreckten Bandabschnitte (10, 12) mit einer zugehörigen Schiene (11, 13) des medial angeordneten Gelenks (14) verbunden sind.

## Claims

1. Knee-joint orthosis for guiding the patella of a patient during the transition from an extended position to a flexed position of the knee joint and vice versa, with a fastening means (1) for fastening to a thigh and a lower leg, with at least one laterally arranged stabilizing element that takes up a flexion of the knee joint and bears on the thigh and lower leg, and with a dimensionally stable guide element (6) that can be held in position by an arrangement of tensioning straps and is arranged to bear laterally on the patella by way of a lateral base part (7) from which medially directed attachments (8) extend proximally and distally of the patella and are secured to strap sections (10, 12) on the orthosis in the area of the thigh and of the lower leg in such a way that a medial tensile force is exerted on the attachments (8), wherein the strap sections (10, 12) are designed with an elasticity and a laterally directed elastic strap (19) allowing a sideways movement of the base part (7) is connected to the base part (7), **characterized in that** the elasticity of the laterally directed strap (19) is greater than the elasticity of the strap sections (10, 12), and that the elasticities of the medially directed strap sections (10, 12), on the one hand, and of the laterally directed strap (19), on the other hand, are chosen such that, during a flexion of the hinge (14) from the extended position, it is the laterally directed strap (19) that first stretches at least quite predominantly, and a stretch limiter (23), provided for the laterally directed strap (19), is active at a predefined flexion limit angle of the knee joint, such that, for a greater flexion of the knee hinge (14), it is the medially directed strap sections (10, 12) that stretch at least predominantly.

2. Knee-joint orthosis according to Claim 1, **characterized in that**, when the flexion limit angle is exceeded, only the medially directed strap sections (10, 12) still stretch.

3. Knee-joint orthosis according to Claim 1 or 2, **characterized in that** the laterally arranged stabilizing element is formed by a hinge (20) with two rails (21, 22) mounted so as to be rotatable relative to each other via the hinge (20), of which a thigh rail (21) is connected to and bears on the fastening means (1) on the thigh, and of which a lower leg rail (22) is connected to and bears on the fastening means (1) on the lower leg.

4. Knee-joint orthosis according to one of Claims 1 to 3, **characterized in that** a hinge (14) arranged on the medial aspect is provided with two rails (11, 13) mounted so as to be rotatable relative to each other via the hinge (14), of which a thigh rail (11) is connected to and bears on the fastening means (1) on the thigh, and of which a lower leg rail (13) is connected to and bears on the fastening means (1) on the lower leg.

5. Knee-joint orthosis according to Claim 4, **characterized in that** the medially directed strap sections (10, 12) are connected to a spring element (17, 18) held in the associated rail (11, 13) of the medial hinge (14), and **in that** the elasticity of the strap sections (10, 12) results from the elasticity of the associated spring element (17, 18).

6. Knee-joint orthosis according to one of Claims 1 to 5, **characterized in that** the fastening means is formed by a sleeve (1) made of elastic material and designed to be pulled over the thigh and lower leg.

7. Knee-joint orthosis according to one of Claims 1 to 6, **characterized in that** the laterally directed strap (19) is fastened to the sleeve (1) by velcro fasteners.

8. Knee-joint orthosis according to one of Claims 3 to 7, **characterized in that** the laterally directed strap (19) is fastened on the laterally arranged hinge (20).

9. Knee-joint orthosis according to one of Claims 1 to 8, **characterized in that** the medially directed strap sections (10, 12) are adjustable in length.

10. Knee-joint orthosis according to one of Claims 1 to 9, **characterized in that** the guide element (6) has a raised edge (9) for bearing on a lateral margin of the patella.

11. Knee-joint orthosis according to one of Claims 1 to 10, **characterized in that** the medially directed strap sections (10, 12) are fastened to the sleeve (1) by velcro fasteners.

12. Knee-joint orthosis according to one of Claims 5 to 10, **characterized in that** the medially directed strap sections (10, 12) are connected to an associated rail (11, 13) of the medially arranged hinge (14).

## Revendications

1. Orthèse de genou pour le guidage de la rotule d'un patient lors du passage d'une position tendue à une position fléchie du genou et inversement, comprenant un dispositif de fixation (1) pour la fixation à une cuisse et à une jambe inférieure, avec au moins un élément de stabilisation agencé latéralement, reprenant une flexion du genou, pour le placement contre la cuisse et la jambe inférieure, et avec un élément de guidage (6) indéformable qui peut être maintenu en position grâce à un agencement de bande de serrage et qui est agencé pour l'ajustement latéral à la rotule au moyen d'une partie de base (7) latérale, à partir de laquelle des appendices (8) orientés médialement s'étendent de façon proximale et distale par rapport à la rotule, et sont fixés à l'orthèse dans la région de la cuisse et de la jambe inférieure par des sections de bande (10, 12) de sorte qu'une tension médiale soit exercée sur les appendices (8), les sections de bande (10, 12) étant formées élastiques, et une bande (19) élastique qui permet un déplacement latéral de la partie de base (7) et qui s'étend latéralement étant reliée à la partie de base (7), **caractérisée en ce que** l'élasticité de la bande (19) s'étendant latéralement est supérieure à l'élasticité des sections de bande (10, 12), et **en ce que** les élasticités des sections de bande (10, 12) s'étendant médialement d'une part, et de la bande (19) s'étendant latéralement d'autre part, sont choisies de sorte que lors d'une flexion de l'articulation (14) à partir de la position tendue, ce soit d'abord, au moins de façon tout à fait prépondérante, la bande (19) s'étendant latéralement qui s'étire, et **en ce qu'**il est prévu un limiteur d'étirement (23) pour la bande (19) s'étendant latéralement, agissant pour un angle limite prédéterminé de flexion du genou, de sorte que pour une flexion plus importante du genou (14), ce soient au moins de façon prépondérante les sections de bande (10, 12) s'étendant médialement qui s'étirent.

2. Orthèse de genou selon la revendication 1, **caractérisée en ce que** lorsque l'angle limite de flexion est dépassé, seules les sections de bande (10, 12) s'étendant médialement s'étirent.

3. Orthèse de genou selon la revendication 1 ou 2, **caractérisée en ce que** l'élément de stabilisation agencé latéralement est formé par une articulation (20) ayant deux barres (21, 22) jointes l'une à l'autre de façon rotative par l'intermédiaire de l'articulation (20), parmi lesquelles une barre de cuisse (21) est fixée adjacente à la cuisse avec le dispositif de fixation (1) et une barre de jambe inférieure (22) est fixée adjacente à la jambe inférieure avec le dispositif de fixation (1).

4. Orthèse de genou selon l'une quelconque des revendications 1 à 3, **caractérisée en ce qu'**il est prévu du côté médial une articulation (14) ayant deux barres (11, 13) jointes l'une à l'autre de façon rotative par l'intermédiaire de l'articulation (14), parmi lesquelles une barre de cuisse (11) est fixée adjacente à la cuisse avec le dispositif de fixation (1) et une barre de jambe inférieure (13) est fixée adjacente à la jambe inférieure avec le dispositif de fixation (1).

5. Orthèse de genou selon la revendication 4, **caractérisée en ce que** les sections de bande (10, 12) s'étendant médialement sont reliées à un élément ressort (17, 18) maintenu dans la barre (11, 13) correspondante de l'articulation médiale (14), et **en ce que** l'élasticité des sections de bande (10, 12) provient de l'élasticité de l'élément ressort (17, 18) correspondant.

6. Orthèse de genou selon l'une quelconque des revendications 1 à 5, **caractérisée en ce que** le dispositif de fixation est formé par un manchon (1) en matériau élastique à enfiler sur la cuisse et la jambe inférieure.

7. Orthèse de genou selon l'une quelconque des revendications 1 à 6, **caractérisée en ce que** la bande (19) s'étendant latéralement est fixée au manchon (1) avec des éléments de fermeture auto-agrippante.

8. Orthèse de genou selon l'une quelconque des revendications 3 à 7, **caractérisée en ce que** la bande (19) s'étendant latéralement est fixée à l'articulation (20) agencée latéralement.

9. Orthèse de genou selon l'une quelconque des revendications 1 à 8, **caractérisée en ce que** les sections de bande (10, 12) s'étendant médialement sont réalisées réglables en longueur.

10. Orthèse de genou selon l'une quelconque des revendications 1 à 9, **caractérisée en ce que** l'élément de guidage (6) est formé par un rebord (9) recourbé vers le haut pour le placement contre un bord latéral de la rotule.

11. Orthèse de genou selon l'une quelconque des revendications 1 à 10, **caractérisée en ce que** les sections de bande (10, 12) s'étendant médialement sont fixées au manchon (1) avec des éléments de fermeture auto-agrippante.

12. Orthèse de genou selon l'une quelconque des revendications 5 à 10, **caractérisée en ce que** les sections de bande (10, 12) s'étendant médialement sont reliées à la barre (11, 13) correspondante de l'articulation (14) agencée médialement.
